# EUROPEAN PATENT APPLICATION

(11) **EP 4 201 956 A1**
(43) Date of publication of application: **28.06.2023**
(21) Application number: 21217585.5
(22) Date of filing: 23.12.2021
(51) Int. Cl.: C07K 16/00, C07K 16/46

(54) **KUTZNERIA ALBIDA (KALBTG) TRANSGLUTAMINASE USED FOR ANTIBODY CONJUGATIONS**

(71) Applicant: F. Hoffmann-La Roche AG, 4070 Basel (CH)
(72) Inventor: The designation of the inventor has not yet been filed
(74) Representative: Skolaut, Alexander

(57) **Abstract**

The disclosure relates to a modified antibody comprising a heavy chain and a light chain, wherein the heavy chain or/and the light chain comprises one or more first recognition site(s) for the transglutaminase from Kutzneria albida (KalbTG). A non-antibody moiety may comprise a therapeutic entity (Antibody Drug conjugate) and optionally a second linker.

## Description

The present invention relates to a modified antibody comprising a heavy chain and a light chain, wherein the heavy chain or/and the light chain comprises one or more first recognition site(s) for the transglutaminase from *Kutzneria albida* (KalbTG) or a functionally active variant thereof. The one or more first recognition site(s) are introduced at one or more selected position(s) within the antibody's heavy chain and/or light chain. The invention further relates to one or more nucleic acids encoding the modified antibody according to the invention as well as a covalent conjugate comprising (i) the modified antibody according to the invention and (ii) one or more non-antibody moieties (payload(s)) covalently conjugated to the one or more first recognition site(s) either directly or via a first linker. In certain embodiments, the non-antibody moiety comprises a therapeutic entity and optionally a second linker. The present invention further relates to a method of covalently conjugating a modified antibody according to the invention to non-antibody moieties. In case the non-antibody moiety comprises a therapeutic entity, the present invention further relates to the conjugate of the modified antibody according to the invention and the therapeutic entity as pharmaceutical composition, for use as a medicament as well as for use in treating a disease.

A pharmaceutical is a chemical substance used to treat, cure or prevent a disease. It can be administered via a number of routes, and many pharmaceuticals can be administered by more than one route. Typical administration routes include without limitation injection as a solution, suspension or emulsion (e.g. intramuscular, intravenous, intraperitoneal, intraocular, intraosseous, subcutaneous or intrathecal), orally, rectally, sublingually or topically. Evidently, a pharmaceutical is usually systemically distributed in the body of the patient and may have adverse effects due to its activity at undesired areas. Other areas may be difficult to reach. Targeted therapy aims to overcome this disadvantage by using pharmaceuticals that are directed to the area in the body, where they are intended to act. Targeted therapies are expected to be more effective than conventional non-targeted forms of treatments and have less side effects.

One way of directing a therapeutic entity to the intended place of action is by conjugating it to an antibody specifically binding at the target cells or tissues. One of the challenges associated with antibody drug conjugates (ADCs), such as antibody-oligonucleotide conjugates (AOCs), is their manufacturing. Most drug-to-antibody couplings make use either of partially reduced interchain disulfide bonds enabling thiol-maleimide chemistry or of lysine functionalization using activated esters or of artificially introduced cysteine residues. The methods result in statistical mixtures of conjugated antibody species with different numbers of drugs attached at different sites. It has been shown that in an ADC with an monomethyl auristatin E payload, the ADC species with a drug-to-antibody ratio (DAR) of 4, 6, or 8, which were demonstrated to be increasingly hydrophobic, were much more prone to aggregation than the DAR2 species (Adem et al., Bioconjugate Chemistry (2014), 25 (4), 656-664).

Therefore, it is desirable to provide ADCs, such as AOCs, for targeted therapy with more precise control over the number and site of the attachment of the therapeutic entities. Thereby homogeneity of the ADC should be increased. Improved site-specific conjugation technologies remain the target of interest of many pharmaceutical companies for their potential use in the preparation of therapeutic ADCs as well as diagnostic antibody-label or antibody-enzyme conjugates.

Microbial transglutaminase from *Streptomyces mobaraensis* has emerged as an inexpensive and easy to use enzyme for protein crosslinking as well as site-specific protein labelling (Ando et al., 2014; Strop et al., 2013).

The discovery of a novel transglutaminase from *Kutzneria albida* and the identification of the respective peptide substrates have been described by Steffen et al. (2017). KalbTG catalyzes the formation of an isopeptide bond between a glutamine side (Gln, Q) chain and a lysine (Lys, K) side chain. YRYRQ (SEQ ID NO: 17) was identified as a KalbTG Gln-containing-motif (Q-tag), while RYESK (SEQ ID NO: 16) was identified as Lys-containing-acceptor-motif (K-tag). KalbTG shows similar efficiency, but improved specificity and developability compared to previously described microbial transglutaminases (mTGs).

The IgG heavy chain C-terminus has been described as a suitable Q-tag insertion site for mTGs for antibody labeling (see, e.g., WO 2021/174091). The present inventors have observed amongst other things increased aggregation and hydrophobicity when conjugating payloads to that site. In order to be able to use KalbTG to prepare antibody-drug conjugates useful as pharmaceuticals, the KalbTG Q-tag has to be introduced at a defined site within the IgG backbone.

In order to circumvent the above-mentioned shortcomings, it was an object of the current invention to identify sites within an IgG molecule for the incorporation of the Q-tag motif resulting in improved properties of the modified antibody with respect to KalbTG-mediated conjugation. The incorporation of the Q-tag should not impair the antibody folding or function or diminish expression yields and should provide the required accessibility for the conjugation as well as therapeutic activity of the therapeutic entity. The successful identification of such sites would allow the incorporation of one or more therapeutic moieties per IgG molecule in a defined stoichiometry as well as a controlled site-specific manner. Thereby more homogeneous conjugate products can be provided, e.g., reducing the required purification and separation efforts and yield molecules with more favorable drug-like properties.

The invention is based, at least in part, on the finding that the Q-tag cannot be incorporated at all sites within an IgG1 antibody with concomitant suitability for conjugation to a payload. The antibody constant domain regions have been screened for conjugation via KalbTG. In this context, KalbTG Q-tags were inserted at surface-exposed inter- and intradomain flexible loops within the human IgG1 heavy and light chain constant regions as well as at the respective heavy and light chain's C-terminus. In total, 9 distinct sites within the IgG1 heavy and light chains were tested; each with an insertion of the KalbTG Q-tag motif within two flexible linkers (GGGSYRYRQGGGS) (SEQ ID NO: 25). Additionally, three antibodies of different binding specificity (mAb 1 to mAb 3) have been tested. These molecules with single-site insertions were assessed for their expression rate. The results are presented in the Examples (see Table 1).

It can be seen that depending on the insertion site expression titer was changed, i.e. reduced or even improved, which was completely unexpected.

The modified antibodies were further tested for conjugation and accessibility of the Q-tag for KalbTG. As payload a small molecule (fluorescent dye) and a small single stranded nucleic acid have been tested. In case the antibody is symmetric, i.e. comprised two identical heavy and light chain pairs, two Q-tags were present per molecule. In case the antibody is asymmetric, i.e. comprised two different heavy and light chain pairs, a single Q-tag is present per molecule. Therefore, a drug-to-antibody ratio (DAR, i.e. number of payload molecules per antibody molecule) of 2 was expected at 100 % conjugation efficiency for the symmetric antibody and a DAR of 1 for the asymmetric antibody. The results for a fluorescent dye in case of mAb 5 are shown in Table 2. The results for a single stranded nucleic acid of 15 nucleotides of length in case of mAb 1 and 2, and 20 nucleotides of length in case of mAb4 are shown in Table 3 (determined by HIC and UV-vis). MAb 1 and 2 were conjugated with a single step method, mAb4 was conjugated in a two-step method (first to the K-tag and then the K-tag to the nucleic acid using click chemistry).

For mAb 2 as an example five different Q-tags, RYGQR (SEQ ID NO: 11), RWRQR (SEQ ID NO: 12), YRQRT (SEQ ID NO: 13), IRQRQ (both Q's can be modified; SEQ ID NO: 14) and FRYRQ (SEQ ID NO: 15), have each been introduced at three different positions, i.e. HC297, HC446 and LC143. The results for expression yield (1 L, µg/mL) and conjugation (DAR; HIC / UV-vis) are shown in the Table 4.

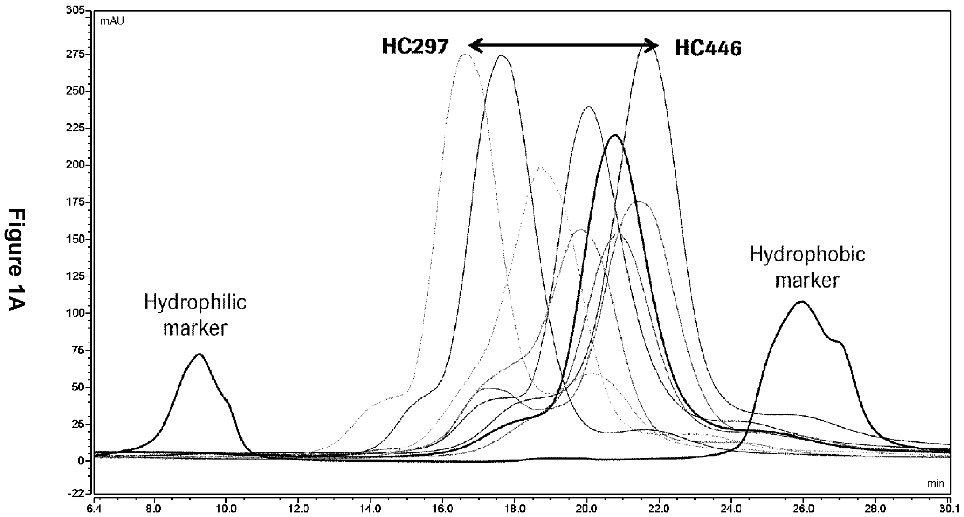

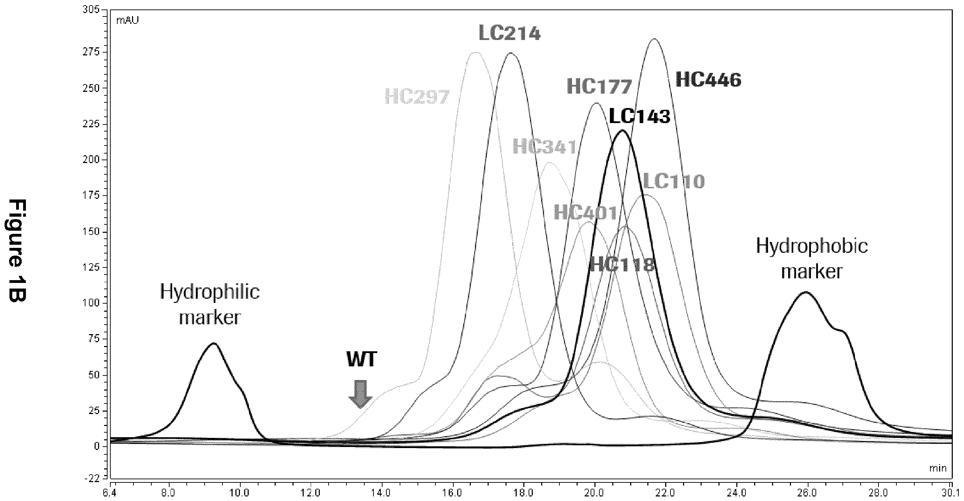

Further, a biophysical characterization of the modified antibody conjugated to a single stranded nucleic acid consisting of 15 nucleotides and 20 nucleotides, respectively, by hydrophobic interaction chromatography (HIC) has been made. This is exemplarily shown for mAb 2 (15 nucleotides) in the Table 5 and Figure 1. The hydrophilic marker had a retention time of 9.25 min. and the hydrophobic marker had a relative retention time of 25.9 min. (mAb 2).

Thus, the inventors have successfully identified a number of KalbTG Q-tag insertion sites spanning the length of the IgG backbone, which do not negatively impact on expression yield and provide enzyme accessibility.

The suitable insertion sites for the Q-tag are at positions 110 (LC110), 143 (LC143) and 214 (LC214) of the light chain and position 118 (HC118), 177 (HC177), 297 (HC297), 341 (HC341) and 401 (HC401) of the heavy chain (the amino acid numbering follows Kabat's EU-numbering scheme).

Accordingly, in a first aspect the present invention relates to a modified antibody comprising a heavy chain and a light chain, wherein the heavy chain or/and the light chain comprises one or more (first) recognition site(s) for the transglutaminase from *Kutzneria albida* (KalbTG) at one or more of the positions selected from position 110 (LC110), position 143 (LC143) and position 214 (LC214) of the light chain and position 118 (HC118), position 177 (HC177), position 297 (HC297) position 341 (HC341) and position 401 (HC401) of the heavy chain (the amino acid numbering follows Kabat's EU-numbering scheme). Accordingly, in the modified antibody of the present invention, the recognition site for KalbTG is positioned internally in the constant regions of the Ig heavy chain polypeptide, i.e. not at any terminus, or/and internally or at the C-terminus of the Ig light chain constant domain. In addition, the modified antibody of the present invention may include a further first recognition site for KalbTG at position 446 (HC446) of the heavy chain, i.e. at the C-terminal end. In the context of the insertion of a recognition site for KalbTG at a position means that the amino acid present at that position in the unmodified sequence is either replaced by the recognition site or the recognition site is preferably inserted after that position in addition.

In certain embodiments, the one or more positions are selected from the group of positions comprising position 214 (LC214) of the light chain and position 118 (HC118), position 177 (HC177), position 297 (HC297) and position 341 (HC341) of the heavy chain (the amino acid numbering follows Kabat's EU-numbering scheme) In one preferred embodiment, the one or more positions are selected from the group of positions comprising position 214 (LC214) of the light chain and position 341 (HC341), position 297 (HC297) and position 177 (HC177) of the heavy chain (the amino acid numbering follows Kabat's EU-numbering scheme).

Likewise, the current invention relates to a modified antibody Fc-region comprising a heavy chain Fc-region, wherein the heavy chain Fc-region comprises one or more (first) recognition site(s) for the transglutaminase from *Kutzneria albida* (KalbTG) at one or more of the positions selected from the group of positions comprising position 118 (HC118), position 177 (HC177), position 297 (HC297), position 341 (HC341) and position 401 (HC401) of the heavy chain (the amino acid numbering follows Kabat's EU-numbering scheme). Accordingly, in the modified antibody Fc-region of the present invention, the recognition site for KalbTG is positioned internally in the constant regions of the Ig heavy chain Fc-region polypeptide, i.e. not at any terminus. In addition, the modified antibody Fc-region of the present invention may include a further first recognition site for KalbTG at position 446 (HC446) of the heavy chain, i.e. at the C-terminal end. In the context of the insertion of a recognition site for KalbTG at a position means that the amino acid present at that position in the unmodified sequence is either replaced by the recognition site or the recognition site is preferably inserted after that position in addition.

In certain embodiments, the one or more positions are selected from the group of positions comprising position 118 (HC118), position 177 (HC177), position 297 (HC297) and position 341 (HC341) of the heavy chain (the amino acid numbering follows Kabat's EU-numbering scheme).

In one preferred embodiment, the one or more positions are selected from the group of positions comprising position 341 (HC341), position 297 (HC297) and position 177 (HC177) of the heavy chain (the amino acid numbering follows Kabat's EU-numbering scheme).

The terms "antibody" and "Ig" are used interchangeably herein. They are used in the broadest sense and include, for example, monoclonal antibodies independent of the binding specificity (including agonist, antagonist, neutralizing antibodies, full length or intact monoclonal antibodies), monovalent antibodies, for example full-length antibodies lacking one Fab), multivalent antibodies, i.e. antibodies that are two- or tetravalent, multispecific antibodies and fragments of full-length antibodies so long as they comprise at least one of the modifications as outlined above.

Naturally occurring antibodies are generated by the assembly of heavy chains only or heavy and light chains. Each heavy chain is composed of four domains: the variable domain (VH) and three constant domains (CH1, CH2, and CH3). The light chain is composed of variable domain (VL) and a constant domain (CL). In case heavy and light chains being present, the light chain pairs with a cognate heavy chain Fab-fragment comprising the VH and CH1 domains. The associated light chain and heavy chain Fab fragment together are denoted as Fab fragment. The heavy chain CH2 and CH3 domains, together denoted as heavy chain Fc-region, dimerize with additional heavy chain CH2 and CH3 domains from a second chain to form the Fc-region. The Fc-region is connected to the Fab-fragment(s) via a flexible hinge region. The hinge region comprises several disulfide bridges that covalently link two heavy chain Fc-regions together. In the Fab-fragment, the light chain and the heavy chain Fab fragment are also connected by one disulfide bridge. However, the connectivity differs among the IgG subclasses. The overall structure of full-length IgGs resembles a Y-shape, with the Fc-region forming the base while the two Fab-fragments form the arms and are available for binding to the antigen.

Within the variable domains reside loops called complementarity determining regions (CDRs). These are mainly responsible for the direct interaction of the antibody with its antigen. Because of the significant variability in the number of amino acids in these CDRs, there are multiple numbering schemes for the variable regions. As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) is used for the constant heavy chain domains (CH1, Hinge, CH2 and CH3, which is herein further clarified by referring to "numbering according to Kabat EU index" in this case).

The term "modified antibody" as used herein, denotes antibodies or antibody Fc-regions according to the invention comprising at least one (artificial) internal Q-tag (at a desired site). Modified antibodies include, but are not limited to, synthetic antibodies, monoclonal antibodies, recombinant antibodies, multispecific antibodies (including bispecific antibodies), humanized antibodies, camelized antibodies, chimeric antibodies, intrabodies, anti-idiotypic (anti-id) antibodies, and functional fragments thereof. The term "functional fragment" refers to a portion of an intact antibody that retains some or all of the binding activity of the antibody from which the fragment is derived. Non-limiting examples of functional fragments of an antibody include Fab-fragments, F(ab')-fragments, F(ab)2-fragments, F(ab')2-fragments, etc. In particular, modified antibodies according to the invention include antibody molecules and immunologically active portions of antibody molecules, for example, antigen-binding domains or molecules that contain an antigen-binding site that binds to the antigen (e.g., one or more complementarity determining regions (CDRs)) as long as the modification according to the invention is present. The modified antibodies provided herein can be of any type (e.g., IgG, IgE, IgM, IgD, IgA, and IgY, in certain embodiments IgG), any class (e.g., IgG1, IgG2, IgG3, IgG4, IgA1, and IgA2, in one preferred embodiment IgG1), or any subclass (e.g. IgG2a and IgG2b). An antibody can be humanized, chimeric and/or affinity matured as well as an antibody from other species, for example, mouse, rabbit and sheep.

In accordance with the present invention, in certain embodiments, the modified antibody comprises at least one heavy chain and at least one light chain. Accordingly, the modified antibody in certain embodiments may have one or two Fab-fragments. In certain embodiments, the modified antibody is a monovalent, monospecific antibody comprising one (full-length) light chain and one (full-length) heavy chain forming a cognate light chain-heavy chain-pair (including one binding site) and one heavy chain Fc-region fragment including a hinge region associated with the Fc-region of the (full-length) heavy chain.

In accordance with the present invention, in certain embodiments, the modified antibody may be based on an IgG1, IgG2, IgG3, or IgG4 antibody, particularly a humanized, mouse, rabbit, or sheep antibody. Exemplary and suitable sequences are given in the following, wherein X^{↓} indicates the insertion sites of Q-tag motifs:
Human-Heavy chain-constant region of IgG1 subclass (G1m1,17 - Caucasian allotype): (SEQ ID NO. 1) 
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRW
QQGNVFSCSVMHEALHNHYTQKSLSLSPG
Human-Heavy chain-constant region of IgG1 subclass (G1m17 - Afroamerican allotype): (SEQ ID NO. 2) 
A↓STKGPSVFPLAPSSKSTSGGTAALGCLVKDYFPEPVTVSWNSGALTSGVHTFPAVLQSS
↓GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQYN↓
STYRVVSVLTVLHQDWLNGKEYKCKVSNKALPAPIEKTISKAKG↓QPREPQVYTLPPSREE
MTKNQVSLTCLVKGFYPSDIAVEWESNGQPENNYKTTPPVLDSD↓GSFFLYSKLTVDKSRW
QQGNVFSCSVMHEALHNHYTQKSLSLSPG↓
 Human-Heavy chain-constant region of IgG2 subclass: (SEQ ID NO. 3) 
ASTKGPSVFPLAPCSRSTSESTAALGCLVKDYFPEPVTVSWNSGALTSGVHTFPAVLQSS
GLYSLSSVVTVPSSNFGTQTYTCNVDHKPSNTKVDKTVERKCCVECPPCPAPPVAGPSVF
LFPPKPKDTLMISRTPEVTCVVVDVSHEDPEVQFNWYVDGVEVHNAKTKPREEQFNSTFR
VVSVLTVVHQDWLNGKEYKCKVSNKGLPAPIEKTISKTKGQPREPQVYTLPPSREEMTKN
QVSLTCLVKGFYPSDIAVEWESNGQPENNYKTTPPMLDSDGSFFLYSKLTVDKSRWQQGN
VFSCSVMHEALHNHYTQKSLSLSPG
Human-Heavy chain-constant reigon of IgG3 subclass: (SEQ ID NO. 4) 
A↓STKGPSVFPLAPCSRSTSGGTAALGCLVKDYFPEPVTVSWNSGALTSGVHTFPAVLQSS
↓GLYSLSSVVTVPSSSLGTQTYTCNVNHKPSNTKVDKRVELKTPLGDTTHTCPRCPEPKSC
DTPPPCPRCPEPKSCDTPPPCPRCPEPKSCDTPPPCPRCPAPELLGGPSVFLFPPKPKDTL
MISRTPEVTCVVVDVSHEDPEVQFKWYVDGVEVHNAKTKPREEQYN↓STFRVVSVLTVLHQ
DWLNGKEYKCKVSNKALPAPIEKTISKTKG↓QPREPQVYTLPPSREEMTKNQVSLTCLVKG
FYPSDIAVEWESSGQPENNYNTTPPMLDSD↓GSFFLYSKLTVDKSRWQQGNIFSCSVMHEA
LHNRFTQKSLSLSPG↓
Human-Heavy chain-constant region of IgG4 subclass: (SEQ ID NO. 5)

ASTKGPSVFPLAPCSRSTSESTAALGCLVKDYFPEPVTVSWNSGALTSGVHTFPAVLQSS
GLYSLSSVVTVPSSSLGTKTYTCNVDHKPSNTKVDKRVSPNMVPHAHHAQAPEFLGGPSV
FLFPPKPKDTLMISRTPEVTCVVVDVSQEDPEVQFNWYVDGVEVHNAKTKPREEQFNSTY
RVVSVLTVLHQDWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSRLTVDKSRWQEG
NVFSCSVMHEALHNHYTQKSLSLSLG

Thus, in some embodiments, the heavy chain constant region is based on a human Ig heavy chain constant region, e.g., human IgG1, human IgG2, human IgG3, or human IgG4 heavy chain constant region. In certain embodiments, the heavy chain constant region comprises at least one Q-tag according to the invention.

In certain embodiments, the human IgG1 heavy chain polypeptide, on which the modified antibody according to the invention may be based, includes a constant region amino acid sequence 75% or more, e.g., 80% or more, 85% or more, 90% or more, 95% or more, 97% or more, and up to 100% identical to the amino acid sequence set forth in SEQ ID NO: 1 or 2. In certain embodiments, the human IgG2 heavy chain polypeptide, on which the modified antibody according to the invention may be based, includes a constant region amino acid sequence 75% or more, e.g., 80% or more, 85% or more, 90% or more, 95% or more, 97% or more, and up to 100% identical to the amino acid sequence set forth in SEQ ID NO: 3. In certain embodiments, the human IgG3 heavy chain polypeptide, on which the modified antibody according to the current invention may be based, includes a constant region amino acid sequence 75% or more, e.g., 80% or more, 85% or more, 90% or more, 95% or more, 97% or more, and up to 100% identical to the amino acid sequence set forth in SEQ ID NO: 4. In certain embodiments, the human IgG4 heavy chain polypeptide, on which the modified antibody according to the current invention may be based, includes a constant region amino acid sequence 75% or more, e.g., 80% or more, 85% or more, 90% or more, 95% or more, 97% or more, and up to 100% identical to the amino acid sequence set forth in SEQ ID NO: 5.

In certain embodiments, the modified antibody according to the invention comprises the following further mutations (numbering according to Kabat):
a) L234A, L235A in both Fc-region polypeptides;
b) P329G in both Fc-region polypeptides;
c) T366W in one Fc-region polypeptide and T366S, L368A, Y407V in the other Fc-region polypeptide;
d) S354C in one Fc-region polypeptide and Y349C in the other Fc-region polypeptide;
e) a) and b);
f) a) and b) and c); or
g) a) and b) and c) and d).

In one preferred embodiment, the modified antibody according to the invention comprises in the first Fc-region polypeptide the mutations L234A, L235A, P329G, T366W and in the second Fc-region polypeptide the mutations L234A, L235A, P329G, T366S, L368A, Y407V. In certain embodiments, the modified antibody further comprises one of the mutation S354C and Y349C in the first Fc-region polypeptide and the other in the second Fc-region polypeptide.

In one preferred embodiment, the human heavy chain polypeptide, on which the modified antibody according to the invention may be based, includes a constant region amino acid sequence 75% or more, e.g., 80% or more, 85% or more, 90% or more, 95% or more, 97% or more, and up to 100% identical to the amino acid sequence set forth in SEQ ID NO: 8 or SEQ ID NO: 9 or SEQ ID NO: 34:
HC177 (SEQ ID NO: 34) 
ASTKGPSVFPLAPSSKSTSGGTAALGCLVKDYFPEPVTVSWNSGALTSGVHTFPAVLQSSG
GGSYRYRQGGGSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG
 or
HC297 (SEQ ID NO: 8) 
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNGGG
SYRYRQGGGSSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG
 or
HC341 (SEQ ID NO: 9)

ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGGGGSYRYRQGGGSQPREPQV
YTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG

In accordance with the present invention, in certain embodiments, the modified antibody may be based on an IgG1, IgG2, IgG3, or IgG4 antibody, particularly a humanized antibody, further comprising a light chain constant domain. Exemplary and suitable sequences are given in the following, wherein X^{↓} indicates the insertion sites of Q-tag motifs:
Human-kappa light chain-constant domain (SEQ ID NO: 6) 

↓
Human-lambda light chain-constant domain (SEQ ID NO: 7)

QPK↓AAPSVTLFPPSSEELQANKATLVCLISDFYPGA↓VTVAWKADSSPVKAGVETTTPSK
QSNNKYAASSYLSLTPEQWKSHRSYSCQVTHEGSTVEKTVAPTEC↓S

In certain embodiments, the human light chain polypeptide, on which the modified antibody according to the invention may be based, includes a constant region amino acid sequence 75% or more, e.g., 80% or more, 85% or more, 90% or more, 95% or more, 97% or more, and up to 100% identical to the amino acid sequence set forth in SEQ ID NO: 6 or 7.

In one preferred embodiment, the human light chain polypeptide, on which the modified antibody according to the invention may be based, includes a constant region amino acid sequence 75% or more, e.g., 80% or more, 85% or more, 90% or more, 95% or more, 97% or more, and up to 100% identical to the amino acid sequence set forth in SEQ ID NO: 10:
LC214 (SEQ ID NO: 10)

RTVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGECGGGSYRYRQGGGS

Preferably, the unmodified light chain constant domain comprises an amino acid sequence that is at least 96 %, 97 %, 98 %, or 99 %, particularly 100 % identical to the amino acid sequence of any of SEQ ID NO: 6 or 7; and/or wherein the unmodified heavy chain constant region comprises an amino acid sequence that is at least 96 %, 97 %, 98 %, or 99 %, particularly 100 % identical to the amino acid sequence of SEQ ID NO: 1 to 5. Also preferably, the light chain constant domain comprises or consists of an amino acid sequence that is at least 96 %, 97 %, 98 %, or 99 %, particularly 100 % identical to the amino acid sequence of SEQ ID NO: 10; and/or wherein the heavy chain constant region comprises or consists of an amino acid sequence that is at least 96 %, 97 %, 98 %, or 99 %, particularly 100 % identical to the amino acid sequence of SEQ ID NO: 8 or 9 or 34.

As detailed above, the modified antibody according to the invention may be a bi- or multispecific antibody. Exemplary embodiments include:
- full-length antibody with domain exchange:
   a multispecific IgG antibody comprising a first Fab fragment and a second Fab fragment, wherein in the first Fab fragment
      a) only the CH1 and CL domains are replaced by each other (i.e. the light chain of the first Fab fragment comprises a VL and a CH1 domain and the heavy chain of the first Fab fragment comprises a VH and a CL domain);
      b) only the VH and VL domains are replaced by each other (i.e. the light chain of the first Fab fragment comprises a VH and a CL domain and the heavy chain of the first Fab fragment comprises a VL and a CH1 domain); or
      c) the CH1 and CL domains are replaced by each other and the VH and VL domains are replaced by each other (i.e. the light chain of the first Fab fragment comprises a VH and a CH1 domain and the heavy chain of the first Fab fragment comprises a VL and a CL domain); and
   wherein the second Fab fragment comprises a light chain comprising a VL and a CL domain, and a heavy chain comprising a VH and a CH1 domain;
   the full length antibody with domain exchange may comprises a first heavy chain including a CH3 domain and a second heavy chain including a CH3 domain, wherein both CH3 domains are engineered in a complementary manner by respective amino acid substitutions, in order to support heterodimerization of the first heavy chain and the modified second heavy chain;
- full-length antibody with domain exchange and additional heavy chain C-terminal binding site:
   a multispecific IgG antibody comprising
      a) one full length antibody comprising two pairs each of a full length antibody light chain and a full length antibody heavy chain, wherein the binding sites formed by each of the pairs of the full length heavy chain and the full length light chain specifically bind to a first antigen, and
      b) one additional Fab fragment, wherein the additional Fab fragment is fused to the C-terminus of one heavy chain of the full length antibody, wherein the binding site of the additional Fab fragment specifically binds to a second antigen,
   wherein the additional Fab fragment specifically binding to the second antigen i) comprises a domain crossover such that a) the light chain variable domain (VL) and the heavy chain variable domain (VH) are replaced by each other, or b) the light chain constant domain (CL) and the heavy chain constant domain (CH1) are replaced by each other, or ii) is a single chain Fab fragment;
- the one-armed single chain format (= one-armed single chain antibody):
   antibody comprising a first binding site that specifically binds to a first epitope or
   antigen and a second binding site that specifically binds to a second epitope or
   antigen, whereby the individual chains are as follows
      - light chain (variable light chain domain + light chain kappa constant domain)
      - combined light/heavy chain (variable light chain domain + light chain constant domain + peptidic linker + variable heavy chain domain + CH1 + Hinge + CH2 + CH3 with knob mutation)
      - heavy chain (variable heavy chain domain + CH1 + Hinge + CH2 + CH3 with hole mutation);
- the two-armed single chain format (= two-armed single chain antibody):
   antibody comprising a first binding site that specifically binds to a first epitope or
   antigen and a second binding site that specifically binds to a second epitope or
   antigen, whereby the individual chains are as follows
      - combined light/heavy chain 1 (variable light chain domain + light chain constant domain + peptidic linker + variable heavy chain domain + CH1 + Hinge + CH2 + CH3 with hole mutation)
      - combined light/heavy chain 2 (variable light chain domain + light chain constant domain + peptidic linker + variable heavy chain domain + CH1 + Hinge + CH2 + CH3 with knob mutation);
- the common light chain bispecific format (= common light chain bispecific antibody):
   antibody comprising a first binding site that specifically binds to a first epitope or
   antigen and a second binding site that specifically binds to a second epitope or
   antigen, whereby the individual chains are as follows
      - light chain (variable light chain domain + light chain constant domain)
      - heavy chain 1 (variable heavy chain domain + CH1 + Hinge + CH2 + CH3 with hole mutation)
      - heavy chain 2 (variable heavy chain domain + CH1 + Hinge + CH2 + CH3 with knob mutation).

As used herein the term "replaced by each other" with respect to corresponding heavy and light chain domains refers to the aforementioned domain crossovers. As such, when CH1 and CL domains are "replaced by each other" it is referred to the domain crossover mentioned under item (i) and the resulting heavy and light chain domain sequence. Accordingly, when VH and VL are "replaced by each other" it is referred to the domain crossover mentioned under item (ii); and when the CH1 and CL domains are "replaced by each other" and the VH and VL domains are "replaced by each other" it is referred to the domain crossover mentioned under item (iii).

Multispecific antibodies also comprise in certain embodiments at least one Fab fragment including either a domain crossover of the CH1 and the CL domains as mentioned under item (i) above, or a domain crossover of the VH and the VL domains as mentioned under item (ii) above, or a domain crossover of the VH-CH1 and the VL-VL domains as mentioned under item (iii) above. In case of multispecific antibodies with domain crossover, the Fabs specifically binding to the same antigen(s) are constructed to be of the same domain sequence. Hence, in case more than one Fab with a domain crossover is contained in the multispecific antibody, said Fab(s) specifically bind to the same antigen.

The term "antigen" refers to a predetermined target to which an antibody can selectively bind. An antigen can be a polypeptide, carbohydrate, nucleic acid, lipid, hapten, or fragment thereof, or other naturally occurring or synthetic compound. In certain embodiments, the antigen is a polypeptide. In another embodiment, the antigen is of therapeutic relevance. In still another embodiment, the antigen is specific for or allows delivery to a specific area in the body, such as to a particular organ or cell type or diseased area. This may be a specific structure on the surface of a cell such as a receptor or a tumor marker. In one preferred embodiment, the antigen is human transferrin receptor.

Preferably, (i) the antibody comprises two pairs each of a heavy chain and a light chain, wherein each of the heavy chains or/and the light chains comprises the one or more first recognition sites, or (ii) the antibody comprises two pairs each of a heavy chain and a light chain, wherein one of the heavy chains or/and the light chains comprises the one or more first recognition sites, or (iii) the antibody comprises one pair of a heavy chain and a light chain and one additional heavy chain Fc-region, wherein the heavy chain or/and the heavy chain Fc-region fragment or the light chain comprises the one or more first recognition sites.

According to the present invention, the modified antibody includes one or more recognition sites for KalbTG in the heavy chain polypeptide and/or the light chain polypeptide. The recognition site includes a motif for KalbTG, wherein KalbTG can catalyze the formation of an isopeptide bond between the modified antibody and a compound (payload) to be coupled to the modified antibody. Typically, the isopeptide bond is formed between a glutamine (Gln) side chain and a lysine (Lys) side chain. Preferably, the modification introduced into an antibody to obtain the modified antibody of the present invention includes the generation of a (artificial) Q-tag, i.e. a Gln containing motif recognized by the KalbTG, within the antibody heavy and/or light chain polypeptide. Suitable Q-tags are disclosed in WO 2017/102759 A1, expressly incorporated by reference herein. The one or more first recognition sites independently of each other comprise or have a Gln-containing motif, especially the sequence RYGQR (SEQ ID NO: 11), RWRQR (SEQ ID NO: 12), YRQRT (SEQ ID NO: 13), IRQRQ (SEQ ID NO: 14), FRYRQ (SEQ ID NO: 15), RYESK (SEQ ID No: 16) or YRYRQ (SEQ ID NO: 17), particularly YRYRQ (SEQ ID NO: 17). The Q-tag may be generated by one or more amino acid modifications, such as substitutions or insertions, preferably insertions. More preferably, the Q-tag is generated by insertion of and/or replacement by an amino acid sequence comprising YRYRQ (SEQ ID NO: 17) or RVRQR (SEQ ID NO: 18), especially YRYRQ (SEQ ID NO: 17). Preferably, the inserted amino acid sequence has a length of 5 to 20 amino acids and comprises YRYRQ (SEQ ID NO: 17) or RVRQR (SEQ ID NO: 18), especially YRYRQ (SEQ ID NO: 17). In a specific embodiment, the insertion is a Q-tag motif without linkers, i.e. the insertion is consisting of YRYRQ (SEQ ID NO: 17) or RVRQR (SEQ ID NO: 18), particularly YRYRQ (SEQ ID NO: 17). The introduction of the one or more recognition sites for KalbTG may be the only modifications in the constant regions of the light and/or heavy chains. Alternatively further modifications may be present, such as a tag for purification (e.g. a His-tag) or other modifications, e.g. for increasing stability or heterodimerization or effector function modification, either alone or in any combination.

As stated above, the one or more first recognition site(s) for KalbTG are inserted into the antibody at specific sites, namely at one or more of the positions selected from position 110 (LC110), 143 (LC143) and 214 (LC214) of the light chain and position 118 (HC118), 177 (HC177), 297 (HC297), 341 (HC341) and 401 (HC401) of the heavy chain, particularly HC177 and/or HC297 and/or LC214. In certain embodiments, the modified antibody of the present invention may include a further recognition site for KalbTG at position 446 (HC446) of the heavy chain, i.e. at the C-terminal end, particularly for coupling to a domain different from that coupled at the first recognition site. Preferably, the one or more first recognition sites are independently of each other at position 177 (HC177) or 297 (HC297) of the heavy chain or at position 214 (LC214) of the light chain.

In certain embodiments, the Q-tag is inserted into the antibody light/heavy chain amino acid sequence via one or two linker(s). The linkers may increase flexibility or allow for conjugation to larger payloads. In certain embodiments, each linker sequence comprises independently of each other between 1 to 20 amino acids, preferably 1 to 10 amino acids, more preferred 1 to 5 amino acids, and more preferred said linker do not interfere essentially with the function of the Q-tag, the KalbTG, the folding of the antibody and the payload to be attached to the modified antibody. The linker may be N- and/or C-terminally attached to the Q-tag. In certain embodiments, the linker amino acids are small amino acids, like glycine or serine. Amino acid linkers and their compositions are known in the art (see, e.g. Chichili et al., Protein Sci. 2013 Feb; 22(2): 153-167). Amino acids glycine, serine, alanine, threonine and glutamate usually constitute amino acids of flexible linkers. Accordingly, the linker(s) may consist primarily or fully of Gly and/or Ser and/or Ala and/or Thr and/or Glu, such as GGGP (SEQ ID NO: 20), ESGS (SEQ ID NO: 21) or APAP (SEQ ID NO: 22). Also, a linker comprising or consisting of KESGSVSSEQLAQFRSLD (SEQ ID NO: 23) or EGKSSGSGSESKST (SEQ ID NO: 24) may be present. In one preferred embodiment, the linkers are consisting independently of each other primarily or fully of Gly and Ser, such as (GlyₘSer)ₙ with m = 1, 2, 3 or 4 and n = 1, 2, 3, 4 or 5, m and n being independently of each other, preferably m=3 and n=1. Preferably, the one or more first recognition sites are inserted into the heavy or/and light chain amino acid sequence via one or two linker(s) at its termini, especially wherein the linker consists primarily or fully of Gly and Ser, such as (Gly-Gly-Gly-Ser)ₙ (SEQ ID NO: 19) with n = 1, 2, 3, 4 or 5, preferably n=1.

If a linker is present, the insertion of the amino acid sequence X1-YRYRQ-X2 (SEQ ID NO: 17) or X1-RVRQR-X2 (SEQ ID NO: 18) into the antibody is preferred. X1 and X2 are independently from each other absent or a linker, particularly linker amino acids. In a specific embodiment, the insertion is a Q-tag motif with two flexible linkers, particularly GGGSYRYRQGGGS (SEQ ID NO: 25) or GGGSRVRQRGGGS (SEQ ID NO: 26), especially GGGSYRYRQGGGS (SEQ ID NO: 25).

An exemplary Fc-region including part of the hinge region with (SEQ ID NO: 32, referred to as 113) and without (SEQ ID NO: 33, referred to as 110) a first recognition site (in bold) has the following sequences:

113: DKTHTCPPCP APEAAGGPSV FLFPPKPKDT LMISRTPEVT CVVVDVSHED PEVKFNWYVD
110: DKTHTCPPCP APEAAGGPSV FLFPPKPKDT LMISRTPEVT CVVVDVSHED PEVKFNWYVD

113: GVEVHNAKTK PREEQYNSTY RVVSVLTVLH QDWLNGKEYK CKVSNKALGA PIEKTISKAK
110: GVEVHNAKTK PREEQYNSTY RVVSVLTVLH QDWLNGKEYK CKVSNKALGA PIEKTISKAK

113: GQPREPQVYT LPPSREEMTK NQVSLTCLVK GFYPSDIAVE WESNGQPENN YKTTPPVLDS
110: GQPREPQVYT LPPSREEMTK NQVSLTCLVK GFYPSDIAVE WESNGQPENN YKTTPPVLDS

113: DGGGSYRYRQ GGGSGSFFLY SKLTVDKSRW QQGNVFSCSV MHEALHNHYT QKSLSLSPGK
110: D......... ....GSFFLY SKLTVDKSRW QQGNVFSCSV MHEALHNHYT QKSLSLSPGK

In a second aspect, the present invention relates to one or more nucleic acids coding for the chains of the modified antibody according to the present invention.

The nucleic acid encoding the modified antibody of the present invention can be isolated or generated in vitro to produce the antibody recombinantly. The nucleic acid may be inserted into a replicable vector for further cloning (amplification of DNA) or for further expression.

"Nucleic acid" has the meaning of comprehensively including DNA (gDNA and cDNA) and RNA molecules, and nucleotides, which are basic structural units in nucleic acids, include not only natural nucleotides, but also analogs with modified sugar or base sites. The sequence of the nucleic acid encoding the heavy and light chain variable regions of the present invention can be modified. Such modifications include additions, deletions, or non-conservative or conservative substitutions of nucleotides as long as the encoded sequence is not changed.

The DNA encoding the modified antibody according to the invention can be isolated or synthesized using conventional procedures (e.g., by using an oligonucleotide probe capable of specifically binding to DNA encoding the heavy and light chains of the antibody).

Many transfer and expression vectors are available. Vector components generally include, but are not limited to, one or more of the following: signal sequence, origin of replication, one or more marker genes, enhancer elements, promoters, and transcription termination sequences.

The term "vector" as used herein refers to a plasmid vector as a means for expressing a gene of interest in a host cell; Cozmid vector; viral vectors such as bacteriophage vectors, adenovirus vectors, retroviral vectors and adeno-associated viral vectors, and the like. The nucleic acid encoding the modified antibody in the vector is operably linked to a promoter and a polyadenylation signal sequence.

"Operatively linked" refers to a functional linkage between a nucleic acid expression control sequence (e.g., a promoter, a signal sequence, or an array of transcriptional regulatory factor binding sites) and another nucleic acid sequence, whereby the control sequence controls the other nucleic acid's transcription and/or translation.

In the case of using eukaryotic cells as a host, promoters derived from the genome of mammalian cells (e.g., metallothionine promoter, β-actin promoter, human hemoglobin promoter, and human muscle creatine promoter) or mammalian promoters derived from animal viruses (e.g., adenovirus late promoter, vaccinia virus 7.5K promoter, SV40 promoter, cytomegalovirus (CMV) promoter, tk promoter of HSV, mouse mammary tumor virus (MMTV) promoter, LTR promoter of HIV, Moloney virus promoter, Epstein-Bar virus (EBV) promoter and Loose sacoma virus (RSV) promoter) may be used. In addition, a polyadenylation signal sequence is present after the encoding nucleic acid as a transcription termination sequence.

Cells may be transformed with the aforementioned vectors. The cells used to generate the antibodies of the present invention may be prokaryotic, yeast or higher eukaryotic cells, but are not limited thereto.

However, the greatest interest is in animal cells and examples of useful host cell lines are COS-7, BHK, CHO, CHO-S, CHO-K1, GS-CHO, CHO DXB-11, CHO DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6 , SP2/0, NS-0, U20S, or HT1080, but is not limited thereto. In one preferred embodiment, the host cell is a CHO cell.

In a third aspect, the present invention relates to a covalent conjugate comprising the (i) the modified antibody according to the invention and (ii) one or more non-antibody (payload) domain(s) covalently conjugated to the one or more (first) recognition site(s) for KalbTG or a functionally active variant thereof, wherein the non-antibody domain comprises a second recognition site for KalbTG. In certain embodiments, the non-antibody domain is a therapeutic moiety comprising a therapeutic entity and optionally a second linker.

As detailed above, the modified antibody of the present invention is provided for conjugating one or more payloads, such as, e.g., therapeutic moieties, specifically to one or more internal site(s) of the antibody with the aid of KalbTG. Thereby, an ADC is obtained, which may be used in the targeted therapy. The conjugate is capable of binding to a target of interest and thereby transports the payload, e.g., the therapeutic moiety, to the intended area in the body. In one preferred embodiment, the modified antibody recognizes a target and binds thereto with its complementarity determining regions (CDRs), particularly wherein the target is a biomolecule present on a cell.

As detailed above, it may be desirable to target a therapeutic moiety to a specific area in a patient's body. This may improve *in vivo* distribution and reduce adverse side effects. Evidently, it may be intended to deliver a therapeutic moiety to an area which is otherwise hard to reach. As an example, it may be envisioned to direct a therapeutic moiety into the brain. Due to the blood-brain-barrier it is difficult to deliver a "naked" therapeutic moiety thereto, if not administered directly into the brain, especially in case the therapeutic moiety exceeds certain size limitations. Therapeutic approaches aiding in overcoming the blood-brain-barrier and transporting the therapeutic moiety into the brain are evidently of advantage. Markedly, the same approach may be used to direct a therapeutic to another area in the body.

In the present invention, the molecular recognition units of antibodies specifically binding to structures in the body are employed in the targeting of the therapeutic moiety. In view of the above, it is evident that the therapeutic entity may be any compound useful in the treatment or prevention of the disease of interest, especially a compound, which is to be delivered to a specific area in the body, such as to a particular organ or cell type or diseased area.

The terms "treat", "treating" and "treatment" are meant to include alleviating or abrogating a condition, disorder, or disease, or one or more of the symptoms associated with the condition, disorder, or disease; or alleviating or eradicating the cause(s) of the condition, disorder, or disease itself. The terms "prevent", "preventing" and "prevention" are meant to include a method of delaying and/or precluding the onset of a condition, disorder, or disease, and/or its attendant symptoms; barring a subject from acquiring a condition, disorder, or disease; or reducing a subject's risk of acquiring a condition, disorder, or disease.

For this, a non-antibody payload comprising a therapeutic entity and optionally a second linker is covalently conjugated to the modified antibody of the invention. The therapeutic moiety comprises the active therapeutic entity or a prodrug. Optionally, a second linker may be present. The second linker may be a chemical linker comprising, e.g., alkyl groups or polyethylene groups, or a peptidic linker.

In one preferred embodiment, the therapeutic entity is a nucleic acid, such as RNA, siRNA or ASO (anti-sense oligonucleotide), particularly an ASO comprising LNA nucleotides; and/or the therapeutic entity is a toxin or a small organic molecule or an immune modulator.

In one preferred embodiment, the therapeutic entity is a nucleic acid. The nucleic acid may be for example DNA or RNA or a mixture thereof. The term RNA also includes anti-sense RNAs as well as small interfering RNAs (siRNAs) which is a class of double-stranded, non-coding RNA molecules, typically 20-24 base pairs in length, similar to miRNA, and operating within the RNA interference (RNAi) pathway. It interferes with the expression of specific genes with complementary nucleotide sequences by degrading mRNA after transcription, thereby preventing translation. The nucleic acid may also comprise one or more locked nucleic acid (LNA), which is a modified RNA nucleotide in which the ribose moiety is modified with an extra bridge connecting the 2' oxygen and 4' carbon. The bridge "locks" the ribose in the *3'-endo* (North) conformation. This structure can be attributed to the increased stability against enzymatic degradation; moreover, the structure of LNA has improved specificity and affinity as a monomer or a constituent of an oligonucleotide. The LNA nucleotide can be mixed with DNA or RNA residues in the oligonucleotide or nucleic acid.

Additionally or alternatively, the therapeutic entity may be a small molecule. In the field of pharmacology, a small molecule is of low molecular weight (< 2,500 daltons, particularly < 1,000 daltons). Many small molecule therapeutic entities are small organic molecules. Small organic molecules typically bind specific biological macromolecules and act as an effector, altering the activity or function of the target.

These compounds can be natural (such as primary and secondary metabolites) or artificial (i.e. not naturally occurring); they have a beneficial effect against a disease.

In the present invention, the therapeutic entity is comprised in a non-antibody domain, which is covalently coupled to the modified antibody of the invention, optionally via a second linker. The linker may depend on the intended target and therapeutic entity, as the length, rigidity and chemical composition of the linker may impact the conjugation reaction rates and the stability of the resulting conjugates. In one preferred embodiment, the linker is an alkyl linker or a polyethylene linker or a peptidic linker or a mixture thereof. In certain embodiments, the linker comprises ethylene glycol (PEG) units, such as about 2 to 50 ethylene glycol units. Exemplary linkers include polyethylene glycol linkers (-NH-C(=O)-PEGₙ-NH₂, with n = 2 to 25). In other embodiments, the linker is an aliphatic carbon chain. The linker may comprise an unsubstituted or substituted alkyl, such as an unsubstituted or substituted C₁₋₆ alkyl, wherein the C₁₋₆ alkyl may be substituted with one or more substituents selected from the group consisting of alkoxy, acyl, acyloxy, alkoxycarbonyl, carbonylalkoxy, acylamino, amino, aminoacyl, aminocarbonylamino, aminocarbonyloxy, cycloalkyl, cycloalkenyl, cyano, azido, halo, hydroxyl, nitro, carboxyl, thiol, thioalkyl, alkyl, alkenyl, alkynyl, heterocyclyl, aminosulfonyl, sulfonylamino, sulfonyl and oxo. In still another embodiment, the linker is a peptidic linker, i.e. a linker composed of amino acids.

In one preferred embodiment, the conjugate comprises a modified antibody according to the present invention and one or more therapeutic nucleic acids, such as ASOs, wherein each therapeutic nucleic acid is linked to a single Q-tag via an amide bond to the terminal residue of the Q-tag via a second linker, especially a PEG linker, as defined above.

In certain embodiments, the conjugate has a DAR ranging from about 1 to about 8, from about 1 to about 4, or from about 1 to about 2. In another embodiment, the conjugate has a DAR of about 1, about 2, about 3, about 4, about 5, about 6, about 7, or about 8.

Upon binding of the antibody to the target, the conjugate is transported into the respective cell by endocytosis, the therapeutic entity is released and can act in the intended manner (e.g., treat a disease).

In one preferred embodiment, the modified antibody recognizes one target and said target is a receptor inducing receptor-mediated endocytosis, such as, e.g., the human transferrin receptor 1 (TfR1), the human insulin-like growth factor 1 receptor (IGF-1R), the human low density lipoprotein receptor-related protein 1 (LRP1), or the human low density lipoprotein receptor-related protein 8 (LRP8), particularly TfR1.

For specific receptors, such as, e.g., the TfR1 or IGF-1R, upon binding of the antibody to the target, the conjugate is transported into the respective cell by endocytosis, released from the endosome and exocytosed again from the cell. If the cell is part of a barrier, such as the blood-brain-barrier, thereby transport across the respective barrier is achieved. Thereby, the therapeutic entity is transported to a compartment of the body, which could not have been reached by the therapeutic entity not conjugated to the modified antibody according to the invention.

In one highly preferred conjugate, the modified antibody is capable of binding to a structure allowing crossing the blood-brain barrier, such as the transferrin receptor.

In one preferred embodiment, the modified antibody recognizes one or two target(s) and said one or two targets is/are specific for a specific cell type, such as a tumor marker being specific for a tumor cell, such a breast cancer cell.

In one preferred embodiment, the modified antibody according to the invention is conjugated to a non-antibody domain comprising a therapeutic entity comprising RNA or LNA for treating or preventing a brain disease, such a Parkinson's disease or Alzheimer's disease, and optionally a PEG linker.

In a fourth aspect, the present invention relates to a method of covalently conjugating a modified antibody according to the invention to a therapeutic entity, the method comprising
a) providing the modified antibody according to the invention,
b) providing a non-antibody domain, wherein the non-antibody domain comprises (i) a therapeutic entity, (ii) a second recognition site for KalbTG, especially wherein the second recognition site comprises or has a Lys-containing motif, especially the sequence RYESK (SEQ ID NO: 16), and (iii) optionally a second linker between the therapeutic entity and the second recognition site; and
c) reacting the modified antibody of a) and the non-antibody domain of b) in the presence of KalbTG or a functionally active variant thereof and under conditions conducive to the activity of KalbTG, thereby forming an isopeptide bond between the first and the second recognition site, thus conjugating the modified antibody to the therapeutic entity.

In the first steps of the method, the modified antibody of the present invention and the non-antibody domain are provided. The non-antibody domain comprises (i) a therapeutic entity, (ii) a second recognition site for KalbTG, especially wherein the second recognition site comprises or has a Lys-containing motif, especially the sequence RYESK (SEQ ID NO: 16) (K-tag), and (iii) optionally a second linker between the therapeutic entity and the second recognition site. The domains may be as defined above. The modified antibody and the non-antibody domain are reacted in the presence of KalbTG or a functionally active variant thereof and under conditions conducive to the activity of KalbTG, thereby an isopeptide bond between the first and the second recognition site is formed, thus conjugating the modified antibody to the therapeutic entity. In certain embodiments, in this method according to the present invention, the antibody comprises one or more Q-tag(s), which is/are conjugated using the activity of KalbTG. The non-antibody domain also comprises a second recognition site for KalbTG. In certain embodiments, the non-antibody domain comprises a K-tag having at least 80% sequence identity to the peptide sequence RYESK (SEQ ID NO: 16).

Microbial transglutaminases (mTGs), including KalbTG catalyze the formation of Gln-Lys isopeptide bonds and are widely used for the cross-linking of proteins and peptides in food and biotechnological applications (e.g. to improve the texture of protein-rich foods or in generating antibody-drug conjugates). KalbTG exhibits essentially no cross-reactivity with known mTG substrates or commonly used target proteins, such as antibodies and therefore allows for the specific conjugation at predetermined sites. Accordingly, essentially any payload comprising a second recognition site (K-tag) for KalbTG, especially wherein the second recognition site comprises or has a Lys-containing motif, especially the sequence RYESK (SEQ ID NO: 16), may be coupled to the modified antibody at the one or more first recognition site(s). KalbTG or a functionally active variant thereof may be as defined in Steffen at al. (2017) or WO 2016/100735 A1, both expressly incorporated by reference herein. The functionally active variant may be a transglutaminase having at least 80%, 90%, 95% or 99% sequence identity to the KalbTG of WO 2016/100735 A1 (see SEQ ID NO: 6 therein). Alternatively, the KalbTG or a functionally active variant thereof may be part of a fusion protein additionally comprising a label such as a tag, e.g. for purification purposes.

In certain embodiments, the KalbTG comprises the amino acid sequence

Met His Lys Trp Phe Leu Arg Ala Ala Val Val Ala Ala Val Gly Phe
Gly Leu Pro Thr Leu Ile Ala Thr Thr Ala Gln Ala Ala Ala Val Ala
Ala Pro Thr Pro Arg Ala Pro Leu Ala Pro Pro Leu Ala Glu Asp Arg
Ser Tyr Arg Thr Trp Arg Val Glu Asp Tyr Val Glu Ala Trp Glu Arg
Tyr His Gly Arg Glu Met Thr Glu Asp Glu Arg Glu Asn Leu Ala Arg
Gly Cys Ile Gly Val Thr Val Val Asn Leu Asn Arg Glu Asp Leu Ser
Asn Pro Pro Leu Asn Leu Ser Phe Gly Ser Leu Arg Thr Ala Glu Ala
Val Gln Ala Ala Leu Asn Lys Ile Val Asp Thr His Pro Ser Pro Ala
Gln Tyr Glu Ala Ala Val Ala Lys Asp Pro Ile Leu Lys Arg Leu Lys
Asn Val Val Lys Ala Leu Pro Ser Trp Ile Asp Ser Ala Lys Leu Lys
Ala Ser Ile Phe Ser Lys Arg Phe Tyr Ser Trp Gln Asn Pro Asp Trp
Ser Glu Glu Arg Ala His Thr Thr Tyr Arg Pro Asp Arg Glu Thr Asp
Gln Val Asp Met Ser Thr Tyr Arg Tyr Arg Ala Arg Pro Gly Tyr Val
Asn Phe Asp Tyr Gly Trp Phe Asp Gln Asp Thr Asn Thr Trp Trp His
Ala Asn His Glu Glu Pro Arg Met Val Val Tyr Gln Ser Thr Leu Arg
His Tyr Ser Arg Pro Leu Gln Asp Phe Asp Glu Gln Val Phe Thr Val
Ala Phe Ala Lys Lys Asp
(SEQ ID NO: 31).

In certain embodiments, in the method according to the present invention said coupling is controlled and, for example, achieved in a stoichiometric ratio of non-antibody domain and modified antibody, for example at about 1:1. Multiple conjugation can also be achieved by using more than one first recognition sites on one modified antibody in order to attach two or even multiple payloads to the modified antibody.

In a fifth aspect, the covalent conjugate comprising the modified antibody of the present invention or produced according to the method of the present invention is for use as a medicament, particularly for use in treating a neurological disease or a brain disease, such as Alzheimer's disease or Parkinson's disease or for use in treating cancer, such as breast cancer.

In certain embodiments, the disease is a neurological disease. In certain embodiments, the neurological disease is selected from the group consisting of a neuropathy disorder, a neurodegenerative disease, cancer, an ocular disease disorder, a seizure disorder, a lysosomal storage disease, amyloidosis, a viral or microbial disease, ischemia, a behavioral disorder, CNS inflammation, Alzheimer's Disease, Parkinson's Disease, multiple sclerosis, CD20 positive cancer with brain metastases, and Her2 positive cancer with brain metastases.

In certain embodiments, the neurological disease is selected from the group consisting of a neuropathy disorder, a neurodegenerative disease, cancer, an ocular disease disorder, a seizure disorder, a lysosomal storage disease, amyloidosis, a viral or microbial disease, ischemia, a behavioral disorder, and CNS inflammation.

As detailed above, the conjugates comprising the modified antibody according to the invention may be used as a medicament, particularly in the treatment of a neurological disease or a brain disease, such as Alzheimer's disease or Parkinson's disease, or for use in treating cancer, such as breast cancer.

The conjugate may be encompassed in a composition. Such a composition also referred to as pharmaceutical composition, is a composition intended for use in the pharmaceutical field or as pharmaceutic and refers to a preparation which is in such form as to permit the biological activity of an active ingredient contained therein to be effective, and which contains no additional components which are unacceptably toxic to a subject to which the pharmaceutical composition would be administered. It may optionally contain a pharmaceutically acceptable excipient, diluent or carrier, such as buffer substances, stabilizers or preservative and optionally further active ingredients, especially ingredients known in connection with pharmaceutical compositions.

In general, the nature of the additional ingredient will depend on the particular form of pharmaceutical composition and the mode of administration being employed. Pharmaceutically acceptable carriers enhance or stabilize the composition, or can be used to facilitate preparation of the composition. Such carriers may include, but are not limited to, saline, buffered saline, dextrose, water, glycerol, solvents, dispersion media, antibacterial and antifungal agents, isotonic and absorption delaying agents, and the like that are physiologically compatible as well as combinations thereof. The formulation should suit the mode of administration. For instance, parenteral formulations usually comprise injectable fluids that include pharmaceutically and physiologically acceptable fluids such as water, physiological saline, balanced salt solutions, aqueous dextrose, glycerol or the like as a vehicle. In addition to biologically neutral carriers, pharmaceutical compositions to be administered can contain minor amounts of non-toxic auxiliary substances, such as wetting or emulsifying agents, preservatives, and pH buffering agents and the like.

The pharmaceutical composition may comprise a stabilizer. The term "stabilizer" refers to a substance which protects the composition from adverse conditions, such as those which occur during heating or freezing, and/or prolongs the stability or shelf-life of the conjugate of the invention in a condition or state. Examples of stabilizers include, but are not limited to, sugars, such as sucrose, lactose and mannose; sugar alcohols, such as mannitol; amino acids, such as glycine or glutamic acid; and proteins, such as human serum albumin or gelatin.

Typically, a therapeutically effective dose or efficacious dose of the conjugate is employed in the pharmaceutical compositions of the invention. The amount of conjugate administered can be initially determined based on guidance of a dose and/or dosage regimen of a comparable uncoupled therapeutic. In general, the conjugates can provide for targeted delivery, thus providing for at least one of reduced dose or reduced administrations in a dosage regimen. Thus, the conjugates can provide for reduced dose and/or reduced administration in a dosage regimen relative to the therapeutic prior to being in a conjugate of the present invention. As noted above, because the conjugates can provide for controlled stoichiometry of drug delivery, dosages of conjugates can be calculated based on the number of drug molecules provided on a per antibody-therapeutic entity conjugate basis.

A pharmaceutical composition of the invention may be administered once or several times or on multiple occasions. The frequency of administration of a conjugate can vary depending on any of a variety of factors, e.g., severity of the symptoms, etc. For example, in some embodiments, the conjugate is administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid).

In certain embodiments, the conjugate or pharmaceutical composition of the invention is administered simultaneously with one or more additional compounds. In certain embodiments, the conjugate or pharmaceutical composition of the invention is administered before or after the additional compound(s).

The pharmaceutical composition of the invention may be used as a medicament for treating an individual. The individual is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the individual is a human.

The pharmaceutical compositions including a conjugate described herein can be delivered to a cell, group of cells, tumor, tissue, or subject using delivery technologies known in the art. In general, any suitable method recognized in the art for delivering the conjugate can be adapted for use with the herein described compositions. For example, delivery can be by local administration, (e.g., direct injection, implantation, or topical administering), systemic administration, or subcutaneous, intravenous, intraocular, intraperitoneal, or parenteral routes, including intracranial (e.g., intraventricular, intraparenchymal and intrathecal), or intramuscular administration. The covalent conjugate of the present invention is preferably to be administered intravenously, intramuscularly, or intraarterially, more preferably intravenously. It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are injectable solutions or suspensions and the like.

As detailed above, the conjugate/pharmaceutical composition of the invention is particularly useful in treating a neurological disease or a brain disease, such as Alzheimer's disease or Parkinson's disease. The term "neurological diseases" encompasses, among others, neurodegenerative diseases, neuroinflammatory diseases or seizure disorders, particularly of the brain. Neurodegenerative diseases are characterized by progressive loss of structure or function of neurons, including death of neurons. Many neurodegenerative diseases including Parkinson's, Alzheimer's, Huntington's, Amyotrophic lateral sclerosis and Multiple Sclerosis occur as a result of neurodegenerative processes. There are many parallels between different neurodegenerative disorders including atypical protein assemblies as well as induced cell death. Neurodegeneration can further be found in many different levels of neuronal circuitry ranging from molecular to systemic. The terms "Neurodegenerative diseases" and "Neuroinflammatory diseases" have a partially overlapping scope. Inflammatory responses are a hallmark of neurodegenerative disease and participate, or contribute, through different mechanisms in the neuronal cell death. The tryptophan catabolism along the Kynurenine pathway (KP) represents one of these mechanisms. Seizure disorders are brain disorders which are characterized by abnormal signaling between brain cells. Seizure disorders can affect part of the brain (partial seizures) or the entire brain (generalized seizures). The most prominent seizure disorder is epilepsy. In order to pass the blood-brain barrier a receptor mediating receptor-induced endocytosis may be used as a target for the conjugate. Examples thereof include transferrin receptor 1 (TfR1), insulin-like growth factor 1 receptor (IGF-1R), low density lipoprotein receptor-related protein 1 (LRP1) or low density lipoprotein receptor-related protein 8 (LRP8), particularly TfR1.

Unless defined otherwise, all technical and scientific terms and any acronyms used herein have the same meanings as commonly understood by one of ordinary skill in the art in the field of the disclosure. Although any methods and materials similar or equivalent to those described herein can be used in the practice as presented herein, the specific methods, and materials are described herein.

The invention is not limited to the particular methodology, protocols, and reagents described herein because they may vary. Any methods and materials similar or equivalent to those described herein can be used in the practice of the present invention, the exemplary methods, and materials are described herein. Further, the terminology used herein is for the purpose of describing particular embodiments only and is not intended to limit the scope of the present invention.

As used herein and in the appended claims, the singular forms "a", "an", and "the" include plural reference unless the context clearly dictates otherwise. Similarly, the words "comprise", "contain" and "encompass" are to be interpreted inclusively rather than exclusively. Similarly, the word "or" is intended to include "and" unless the context clearly indicates otherwise. The term "plurality" refers to two or more.

The following Figures, Sequences and Examples are intended to illustrate various embodiments of the invention. As such, the specific modifications discussed are not to be construed as limitations on the scope of the invention. It will be apparent to the person skilled in the art that various equivalents, changes, and modifications may be made without departing from the scope of the invention, and it is thus to be understood that such equivalent embodiments are to be included herein.

### FIGURES

- **Figure 1**: illustrates the hydrophobic interaction chromatography of mAb 2 (HER2) with 9 different Q-tag insertion sites, conjugated to a single stranded nucleic acid with 15 residues.

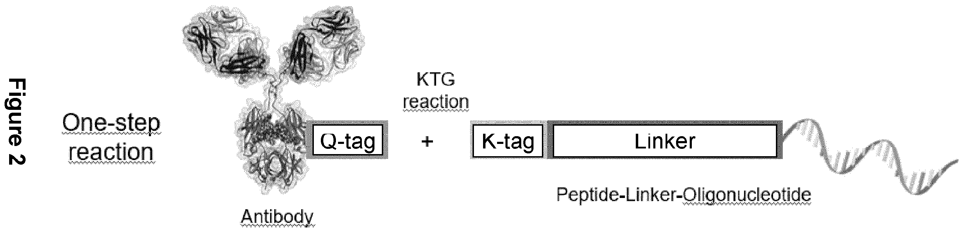

- **Figure 2**: illustrates an exemplary reaction for a single step KalbTG-mediated conjugation of a mAb with nucleic acid payload.

### EXAMPLES

### Example 1:

### Recombinant production of the modified antibody according to the invention

### Gene Synthesis

Desired gene segments were prepared by chemical synthesis and the synthesized gene fragments were cloned into a suitable vector for expression in HEK293 and Expi293 cells by Twist Bioscience (San Francisco, US).

### Expression of the modified antibody in mammalian cells

Antibody production was performed by transient co-transfection of single expression cassette plasmids in HEK293 cells cultivated in F17 Medium (Invitrogen, Carlsbad, CA, USA) or Expi293 cells in Expi293 Expression medium (Thermo Scientific, Waltham, MA, USA), respectively. Transfection was carried out as specified in the manufacturer's instructions with a plasmid ratio of HC:LC expression plasmids = 1:1 in case of symmetric standard IgG1 format, or with a plasmid ratio of HC1:HC2:LC expression plasmids = 1:1:1 in case of asymmetric knob-into-hole formats. Cell culture supernatants were harvested seven days after transfection. Supernatants were stored at reduced temperature (e.g. -20 °C).

### Quantification of protein titer

The protein titer of supernatant samples was determined by affinity chromatography using a POROS A 20 µm column, 2.1 x 30 mm (Life Technologies, Carlsbad, CA, USA) on a High Performance Liquid Chromatography system (Ultimate 3000 HPLC system, Thermo Scientific, Waltham, MA, USA). The supernatant was loaded onto the column equilibrated with 0.2 M Na₂HPO₄, pH 7.4, followed by elution with 0.1 M citric acid, 0.2 M NaCl, pH 2.5. Titers were quantified by measuring absorption at 280 nm, and subsequently calculating the protein concentration by comparing the elution peak area (under the curve) of the analyte with a reference standard curve.

### Purification of modified antibody from mammalian culture supernatant

Antibodies in the culture supernatant were captured by Protein A affinity chromatography using a Mab Select SuRe column (GE Healthcare, Chicago, IL, USA), equilibrated with PBS buffer, pH 7.4. Unbound protein was removed by washing with equilibration buffer. The modified antibody was eluted with 50 mM citrate, pH 3.0 and the pH of the eluate was immediately adjusted to pH 7.5 by addition of 2 M Tris, pH 9.0. Size exclusion chromatography using a Superdex 200^{™} column (GE Healthcare, Chicago, IL, USA) in 20 mM Histidine, 140 mM NaCl, pH 6.0 was performed as second purification step. Purified modified antibodies were stored at -80°C.

### Purification of antibodies using Mid-scale Automation (MilAn)

Antibodies were purified in one step using Protein A affinity chromatography as described above, using MabSelectSure-Sepharose (Cytiva, Marlborough, MA, USA) on a liquid handling system (Tecan, Männedorf, Switzerland), equipped with columns from Repligen (Waltham, MA, USA). Equilibration, sample loading, and washing steps were performed as described, and 25 mM citrate, pH 3.0 was used to elute the antibodies from the column. The eluted antibody fractions were neutralized with 1.5 M Tris, pH 7.5 and the concentration was determined by measuring the optical density (OD) at 280 nm.

### Overview of exemplary antibodies

### Antibody 110:

**Description:**
anti-HER2 antibody based on IgG1 subclass with P329G/L234A/L235A mutation; Q-tag insertion into HC after aa177 (EU numbering); Q-tag and spacer sequence: GGGSYRYRQGGGS (SEQ ID NO: 25)
**Heavy Chain Constant Region Sequence** 
ASTKGPSVFPLAPSSKSTSGGTAALGCLVKDYFPEPVTVSWNSGALTSGVHTFPAV
LQSSGGGSYRYRQGGGSGLYSLSSVVTVPSSSLGTQTYICNVNHKPSNTKVDKKVE
PKSCDKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKAL
GAPIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHYTQKS
LSLSPGK (SEQ ID NO. 27)
**Light Chain Constant Domain Sequence**


 (SEQ
ID NO. 29)

### Antibody 113:

### Description:

Anti-HER2 antibody based on IgG1 subclass with P329G/L234A/L235A mutation; Q-tag insertion into HC after aa401 (EU numbering); Q-tag and spacer sequence: GGGSYRYRQGGGS (SEQ ID NO: 25)
**Heavy Chain Constant Region Sequence** 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPC
PAPEAAGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALGAPIEKTISKAKG
QPREPQVYTLPPSREEMTKNQVSLTCLVKGFYPSDIAVEWESNGQPENNYKTTPPV
LDSDGGGSYRYRQGGGSGSFFLYSKLTVDKSRWQQGNVFSCSVMHEALHNHYTQKS
LSLSPGK (SEQ ID NO. 28)
**Light Chain Constant Domain Sequence**


 (SEQ
ID NO. 30)

**Table 1: Expression yield of different antibodies with Q-tags inserted at various positions.**

| yield of at scale [mg] | mAb 1 | | mAb 2 | | mAb 3 | |
|---|---|---|---|---|---|---|
| | (CD163) | | (HER2) | | (LeY) | |
| → | 24 mL | 1L | 24 mL | 1L | 24 mL | 1L |
| position of Q-tag ↓ | | | | | | |
| wt without Q-tag (reference) | 4.7 | 196 | 4.3 | 179 | 0.4 | 17 |
| HC118 | 7.5 | 313 | 2.5 | 104 | 1.0 | 42 |
| HC177 | 7.3 | 304 | 2.6 | 108 | 1.5 | 63 |
| HC297 | 7.7 | 321 | 2.9 | 121 | 1.6 | 67 |
| HC341 | 6.4 | 267 | 3.4 | 142 | 1.7 | 71 |
| HC401 | 4.5 | 188 | 2.7 | 113 | 1.0 | 42 |
| HC446 | 4.3 | 179 | 2.8 | 117 | 1.3 | 54 |
| LC110 | 1.7 | 70.8 | 3.0 | 125 | 0.4 | 17 |
| LC143 | 0.7 | 29.2 | 1.1 | 46 | 0.4 | 17 |
| LC214 | 3.3 | 138 | 2.5 | 104 | 0.5 | 21 |

The expression of the modified antibody is influenced by the position of the introduced Q-tag. Introduction after position LC110, LC214, HC118, HC177, HC297, HC341 and HC401 resulted in the highest yields.

### Example 2:

### KalbTG conjugation of the modified antibody according to the invention to a fluorescent dye

### Conjugation using KalbTG

Purified antibodies containing a Q-tag were transferred into conjugation buffer (histidine buffer comprising NaCl, pH 8.5) via dialysis. For the KalbTG reaction, the antibody was mixed with K-tagged small molecule (fluorescent dye, 10x molar excess) and KalbTG was added (molar ratio mAb:KalbTG > 100:1). The reaction mixture was incubated at 37 °C with shaking , and the reaction was subsequently quenched by adding 10 mM ammonium sulfate to the solution. To remove unconjugated payload and residual enzyme, the conjugated modified antibody was purified by size exclusion chromatography using a Superdex 200^{™} column (GE Healthcare, Chicago, IL, USA) in PBS pH 7.5. The purified conjugates were stored at -80 °C.

### Analysis of conjugates

Protein quantification was performed with a Nanodrop spectrophotometer (ThermoFisher Scientific, Waltham, MA, USA). In addition, qualitative DAR measurements were carried out by Hydrophobic interaction chromatography as described in Example 4 below. The purity of the conjugates was analyzed by CE-SDS under denaturing and reducing conditions using a Caliper LabChip^{®} GXII Touch^{™} protein characterization system according to the manufacturer's instructions (Perkin Elmer, Waltham, MA, USA).

Aggregate content was determined by SEC using a TSKgel UP-SW3000 analytical size-exclusion column (Tosoh Bioscience, Griesheim, Germany), equilibrated with 0.2 M K₂HPO₄/KH₂PO₄, 0.25 M KCI, pH 6.2, on a High Performance Liquid Chromatography system (Ultimate 3000 HPLC system, Thermo Fisher Scientific, Waltham, MA, USA).

The identity of the conjugates was confirmed by ESI-Q-ToF-MS (Bruker maXis 433, Bruker, Billerica, MA, USA). For MS analysis, the samples were deglycosylated using N-glycosidase F (Roche, Basel, Switzerland) and subsequently desalted into 2% formic acid, 40% acetonitrile.

**Table 2: Conjugation efficiency of a fluorescent dye to antibody mAb 5 with Q-tags at various positions**

| conjugation efficiency [DAR] | mAb 5 (muTfR) |
|---|---|
| → | |
| position of Q-tag ↓ | DAR (HIC) |
| HC118 | 1.8 |
| HC177 | 1.8 |
| HC297 | 1.7 |
| HC341 | 1.8 |
| HC401 | 2.0 |
| LC110 | 1.4 |
| LC143 | 1.3 |
| LC214 | 1.5 |

### Example 3:

### KalbTG conjugation of the modified antibody according to the invention to a nucleic acid

### Synthesis of antisense oligonucleotides

Single-stranded LNA oligonucleotides were synthesized using standard phosphoramidite chemistry. DNA and LNA phosphoramidites and all standard reagents were purchased from Merck KGaA (Darmstadt, Germany). K-tag peptides were custom synthesized by Schafer-N Aps (Copenhagen, Denmark) and Biosyntan (Berlin, Germany).

Oligonucleotides were synthesized on NittoPhase HL UnyLinker 350 support (Kinovate, Oceanside, CA) on an ÄKTA Oligopilot (GE Healthcare, Brondby, Denmark) at 130 mmol scale. After synthesis, the oligonucleotides were cleaved from the support overnight. The oligonucleotides were purified by ion exchange chromatography and desalted using a Millipore membrane. After lyophilization, the compounds were finally characterized by liquid chromatography-mass spectrometry (reverse phase and electrospray ionization-mass spectrometry).

The oligonucleotides were conjugated either to the respective linker for conjugating to the K-tag (1-step reaction) or the acceptor part for Click chemistry conjugation (2-step reaction). The conjugates were then subjected directly to purification by reversed phase HPLC as described below.

After precipitation of the linker oligonucleotide by 2% lithium perchlorate in acetone, the resulting precipitate was washed with acetone, dried under vacuum and redissolved in PBS. 1.5 equivalents of K-tag peptide was dissolved in PBS and added. After 1 hour at room temperature, the reaction mixture was subjected directly to purification by reversed phase HPLC.

Both reactions above were purified by reversed phase HPLC on a Waters XBridge Peptide BEH C18 OBD Prep Column, 300 Å, 10 µm, 10 mm X 150 mm using 0.1 M ammonium acetate and acetonitrile as eluent. Pooled fractions were lyophilized, redissolved in water and pH adjusted to pH 7.0 with aqueous NaOH. After a final lyophilization, the compounds were finally characterized by liquid chromatography-mass spectrometry (reverse phase and electrospray ionization-mass spectrometry).

### Enzymatic conjugation of LNA-ASOs to antibodies with Q-tags

### 1-Step conjugation using KalbTG

Purified antibodies containing a Q-tag were transferred into conjugation buffer (histidine buffer comprising about 150 mM chloride ions,pH 7.5) via dialysis. For the KalbTG reaction, the antibody was mixed with excess of K-tagged oligonucleotide and KalbTG (Roche Diagnostics, Mannheim, Germany) was added. The reaction mixture was incubated at 37 °C with shaking, and the reaction was subsequently quenched by adding 10 mM ammonium sulfate to the solution. To remove unconjugated payload and residual enzyme, the conjugated modified antibody was purified by size exclusion chromatography using a Superdex 200^{™} column (GE Healthcare, Chicago, IL, USA) in PBS, 250 mM arginine, pH 7.5. The purified conjugates were stored at -80 °C.

### 2-Step conjugation using KalbTG and Click chemistry

Purified antibodies containing a Q-tag were transferred into conjugation buffer (histidine buffer comprising NaCl, pH 8.5) via dialysis. For the KalbTG reaction, the antibody was mixed with excess of K-tagged linker comprising the first part of Click conjugation (10x molar excess) and KTG was added. The reaction mixture was incubated at 37 °C with shaking, and the reaction was subsequently quenched by adding 10 mM ammonium sulfate to the solution. To remove unconjugated linker and residual enzyme, the conjugated modified antibody was purified by size exclusion chromatography using a Superdex 200^{™} column (GE Healthcare, Chicago, IL, USA) in PBS, 250 mM arginine, pH 7.5. The purified antibody-linker conjugate was added to excess of the oligonucletiode conjugated to the respective other part of Click conjugation in PBS, 250 mM arginine, pH 7.5 and the reaction mixture was incubated over night at room temperature with shaking. The antibody-oligonucleotide conjugate was purified by size exclusion chromatography as described above and purified conjugates were stored at -80 °C.

### Analysis of conjugates

Quantification of oligonucleotide conjugates was performed by UV/Vis spectrometry at 260, 280, and 350 nm with the SoloVPE system (C Technologies, Bridgwater, NJ, USA). Conjugate concentrations and a quantitative Drug-to-Antibody Ratio (DAR) were calculated with the Lambert-Beer equation. In addition, qualitative DAR measurements were carried out by Hydrophobic interaction chromatography as described in Example 4 below. The purity of the conjugates was analyzed by CE-SDS under denaturing and reducing conditions using a Caliper LabChip^{®} GXII Touch^{™} protein characterization system (Perkin Elmer, Waltham, MA, USA). Aggregate content was determined by SEC using a TSKgel UP-SW3000 analytical size-exclusion column (Tosoh Bioscience, Griesheim, Germany), equilibrated with 0.2 M K₂HPO₄/KH₂PO₄, 0.25 M KCI, pH 6.2, on a High Performance Liquid Chromatography system (Ultimate 3000 HPLC system, Thermo Fisher Scientific, Waltham, MA, USA). The identity of the conjugates was confirmed by ESI-Q-ToF-MS (Bruker maXis 433, Bruker, Billerica, MA, USA). For MS analysis, the samples were deglycosylated using N-glycosidase F (Roche, Basel, Switzerland) and subsequently desalted into 2 % formic acid, 40 % acetonitrile.

**Table 3: Conjugation efficiency of a nucleic acid consisting of 15 or 20 nucleotides to different antibodies with Q-tags at various positions**

| conjugation efficiency [DAR2] | mAb 1 (CD163) | mAb 2 (HER2) | | mAb 4 (TfR) |
|---|---|---|---|---|
| → | | | | |
| position of Q-tag | | | | |
| ↓ | (HIC) | (HIC) | (UV-vis) | (HIC) |
| HC118 | 1.5 | 1.9 | 1.8 | n.d. |
| HC177 | 2.0 | 1.9 | 1.8 | n.d. |
| HC297 | | | | |
| asymmetric | n.d. | n.d. | n.d. | 0.9 |
| symmetric | 2.0 | 1.9 | 1.7 | 1.9 |
| HC341 | 2.0 | 1.8 | 1.8 | n.d. |
| HC401 | 1.9 | 1.8 | 1.7 | n.d. |
| HC446 | | | | |
| asymmetric | n.d. | n.d. | n.d. | 0.9 |
| symmetric | 2.0 | 1.9 | 1.8 | 1.8 |
| LC110 | 2.0 | 1.8 | 1.7 | n.d. |
| LC143 | 1.9 | 1.9 | 1.7 | n.d. |
| LC214 | | | | |
| asymmetric | n.d. | n.d. | n.d. | 1.0 |
| symmetric | 2.0 | 1.8 | 1.7 | n.d. |

| | | | | |
|---|---|---|---|---|
| n.d. = note determined | | | | |

The conjugation of the modified antibody is influenced by the position of the introduced Q-tag. Introduction after position LC110, LC143, LC214, HC118, HC177, HC297, and HC341 resulted in the best conjugation efficiencies.

**Table 4: Expression yields and conjugation efficiencies for modified antibodies with different Q-tags**

| position of Q-tag | HC297 | | HC446 | | LC143 | |
|---|---|---|---|---|---|---|
| → | | | | | | |
| Q-tag sequence ↓ | yield | conjugation efficiency | yield | conjugation efficiency | yield | conjugation efficiency |
| RYGQR (SEQ ID NO: 11) | 90 | 1.6/1.5 | 144 | 1.8/1.7 | 42 | 0.7/0.6 |
| RWRQR (SEQ ID NO: 12) | 136 | 1.7/1.6 | 114 | 1.8/1.7 | 12.2 | 1.1/0.8 |
| YRQRT (SEQ ID NO: 13) | 102 | 1.7/1.7 | 0 | n.d. | 28 | 1.8/1.7 |
| IRQRQ (SEQ ID NO: 14) | 132 | 3.1/3.1 | 124 | 2.8/2.8 | 44 | 2.6/2.2 |
| FRYRQ (SEQ ID NO: 15) | 136 | 1.6/1.7 | 62 | 1.5/1.3 | 22 | 1.7/1.7 |

| | | | | | | |
|---|---|---|---|---|---|---|
| n.d. = not determined | | | | | | |

### Example 4:

### Hydrophobic Interaction Chromatography of KalbTG conjugated modified antibody according to the invention

Hydrophobic Interaction Chromatography (HIC) was performed on a High Performance Liquid Chromatography system (Ultimate 3000 HPLC system, Thermo Fisher Scientific, Waltham, MA, USA) using a TSKgel Butyl-NPR column (2.5 µm, 4.6 x 35 mm, TOSOH Bioscience, Tokyo, Japan) with a flow rate of 1 mL/min. The column was equilibrated with Eluent A (20 mM Na₂HPO₄ dihydrate, 1.5 M (NH₄)₂SO₄, pH 7.0) and 60 µg of each sample was loaded onto the column. Subsequently, a gradient between Eluent A and Eluent B (20 mM Na₂HPO₄ dihydrate, 25 % (v/v) isopropanol, pH 7.0) was applied.

**Gradient:**

| | |
|---|---|
| 0 min | 5 % B |
| 0-30 min | 5 % B -> 80 % B |
| 30 - 34 min | 80 % B -> 100 % B |
| 34 - 44 min | 100 % B |
| 45 - 55 min | 0 % B |

The elution profile was obtained by continuous measurement of the absorbance at 280 nm. Drug to antibody ratios (DAR) were determined by peak integration using Chromeleon 7.2 (Thermo Fisher Scientific, Waltham, MA, USA).

An exemplary result is shown in Figure 1.

**Table 5: Retention times of modified antibodies conjugated to a nucleic acid consisting of 15 nucleotides (mAb 2) and 20 nucleotides (mAb 4).**

| relative retention time | mAb 2 (HER2) | | mAb 4 (TfR) | |
|---|---|---|---|---|
| → | not conjugated | conjugated to nucleic acid | not conjugated | conjugated to nucleic acid |
| position of Q-tag ↓ | | | | |
| mAb 2 (wt) (reference) | 0.246 | n.d. | 0.023 | n.d. |
| HC118 | 0.311 | 0.685 | n.d. | n.d. |
| HC177 | 0.221 | 0.647 | n.d. | n.d. |
| HC297 asymmetric symmetric | n.d. 0.233 | n.d. 0.446 | 0.023 0.070 | 0.472 0.670 |
| HC341 | 0.269 | 0.566 | n.d. | n.d. |
| HC401 | 0.247 | 0.634 | n.d. | n.d. |
| HC446 asymmetric symmetric | n.d. 0.215 | n.d. 0.745 | 0.015 0.001 | 0.845 0.994 |
| LC110 | 0.402 | 0.732 | n.d. | n.d. |
| LC143 | 0.291 | 0.691 | n.d. | n.d. |
| LC214 | 0.246 | 0.501 | 0.020 | 0.556 |

The hydrophilic marker had a retention time of 9.25 min. and the hydrophobic marker had a relative retention time of 25.9 min. (mAb 2) or of 9.00 min. and 24.7 min. (mAb 4), respectively.

The hydrophobicity of the conjugate is influenced by the position of the introduced Q-tag. Introduction after position LC110, LC214 and HC297 resulted at most in a 2-fold change of non-conjugated to conjugated modified antibody, whereas HC177 had the smallest relative retention time of all tested the internal insertion sites.

### REFERENCES

- Adem et al., Bioconjugate Chemistry (2014), 25 (4), 656- 664.
- Agarwal, P. & Bertozzi, C. R. Site-specific antibody-drug conjugates: the nexus of bioorthogonal chemistry, protein engineering, and drug development. Bioconjug Chem 26, 176-192, doi:10.1021/bc5004982 (2015).
- Ando, H. et al. Purification and Characteristics of a Novel Transglutaminase Derived from Microorganisms. Agricultural and Biological Chemistry 53, 2613-2617, doi:10.1080/00021369.1989.10869735 (2014).
- Chichili et al. Linkers in the structural biology of protein-protein interactions Protein Sci. 2013 Feb; 22(2): 153-167
- Steffen, W. et al. Discovery of a microbial transglutaminase enabling highly site-specific labeling of proteins. J Biol Chem, doi:10.1074/jbc.M117.797811 (2017).
- Strop, P. et al. Location matters: site of conjugation modulates stability and pharmacokinetics of antibody drug conjugates. Chem Biol 20, 161-167, doi:10.1016/j.chembiol.2013.01.010 (2013).
- WO 2021/174091
- WO 2017/102759
- Zhou, Q. & Kim, J. Advances in the Development of Site-Specific Antibody-Drug Conjugation. (2015).

## Claims

1. A modified antibody comprising a heavy chain or/and a light chain, wherein the heavy chain or/and the light chain comprises one or more first recognition site(s) for the transglutaminase from *Kutzneria albida* (KalbTG) independently of each other at one or more of the positions selected from position 110 (LC110), position 143 (LC143) and position 214 (LC214) of the light chain and position 118 (HC118), position 177 (HC177), position 297 (HC297), position 341 (HC341) and position 401 (HC401) of the heavy chain.

2. The modified antibody of claim 1, wherein (i) the antibody comprises two pairs each of a heavy chain and a light chain, wherein each of the heavy chains or/and the light chains comprises the one or more first recognition sites, or (ii) the antibody comprises one pair of a heavy chain and a light chain and one additional heavy chain Fc-region, wherein the heavy chain and the heavy chain Fc-region fragment or the light chain comprises the one or more first recognition sites.

3. The modified antibody of claim 1 or 2, wherein the one or more first recognition sites are independently of each other at position 177 (HC177) or 297 (HC297) of the heavy chain or at position 214 (LC214) of the light chain.

4. The modified antibody of any one of claims 1 to 3, wherein the one or more first recognition sites independently of each other comprise or have a Gln-containing motif, especially the sequence RYGQR (SEQ ID NO: 11), RWRQR (SEQ ID NO: 12), YRQRT (SEQ ID NO: 13), IRQRQ (SEQ ID NO: 14), FRYRQ (SEQ ID NO: 15), RYESK (SEQ ID No: 16) or YRYRQ (SEQ ID NO: 17), particularly YRYRQ (SEQ ID NO: 17).

5. The modified antibody of any one of claims 1 to 4, wherein the one or more first recognition sites are inserted into the heavy or/and light chain amino acid sequence via one or two linker(s) at its termini, especially wherein the linker consists primarily or fully of Gly and Ser, such as (Gly-Gly-Gly-Ser)ₙ (SEQ ID NO: 19) with n = 1, 2, 3, 4 or 5, preferably n=1.

6. The modified antibody of any one of claims 1 to 5, wherein the unmodified light chain constant domain comprises an amino acid sequence that is at least 96 %, 97 %, 98 %, or 99 %, particularly 100 % identical to the amino acid sequence of any of SEQ ID NO: 6 or 7; and/or wherein the unmodified heavy chain constant region comprises an amino acid sequence that is at least 96 %, 97 %, 98 %, or 99 %, particularly 100 % identical to the amino acid sequence of SEQ ID NO: 1 to 5.

7. The modified antibody of any one of claims 1 to 6, wherein the light chain constant domain comprises or consists of an amino acid sequence that is at least 96 %, 97 %, 98 %, or 99 %, particularly 100 % identical to the amino acid sequence of SEQ ID NO: 10; and/or wherein the heavy chain constant region comprises or consists of an amino acid sequence that is at least 96 %, 97 %, 98 %, or 99 %, particularly 100 % identical to the amino acid sequence of SEQ ID NO: 8 or 9 or 34.

8. A nucleic acid coding for the modified antibody of any one of claims 1 to 7.

9. A covalent conjugate comprising the (i) the modified antibody of any one of claims 1 to 7 and (ii) one or more non-antibody (payload) domain(s) covalently conjugated to the one or more first recognition site(s) for KalbTG or a functionally active variant thereof, wherein the non-antibody domain comprises a second recognition site for KalbTG, a therapeutic entity and optionally a second linker.

10. The covalent conjugate of claim 9, wherein
a) the modified antibody recognizes a target and binds thereto with its complementarity determining regions (CDRs), particularly wherein the target is a biomolecule present on a cell; and/or
b) the second linker is an alkyl linker or a polyethylene linker or a peptidic linker; and/or
c) the therapeutic entity is a nucleic acid, such as RNA, siRNA or anti-sense oligonucleotide (ASO), LNA, particularly an ASO comprising LNA nucleotides; and/or
d) the therapeutic entity is a toxin or a small organic molecule or an immune modulator.

11. The modified antibody according to any one of claims 1 to 7 or the covalent conjugate of claim 9 or 10,
a) wherein the modified antibody recognizes one target and said target is a receptor inducing receptor-mediated endocytosis, such as transferrin receptor protein 1 (TfR1), insulin-like growth factor 1 receptor (IGF1R), low density lipoprotein receptor-related protein 1 (LRP1) or low density lipoprotein receptor-related protein 8 (LRP8), particularly TfR1; and/or
b) wherein modified antibody recognizes one or two target(s) and said one or two targets is/are specific for a specific cell type, such as a tumor marker being specific for a tumor cell, such a breast cancer cell.

12. A method of covalently conjugating a modified antibody to a therapeutic moiety, the method comprising
a) providing the modified antibody of any one of claims 1 to 7 or 11,
b) providing a non-antibody domain, wherein the non-antibody domain comprises (i) a therapeutic entity, (ii) a second recognition site for KalbTG, especially wherein the second recognition site comprises or has a Lys-containing motif, especially the sequence RYESK (SEQ ID NO: 16), and (iii) optionally a second linker between the therapeutic entity and the second recognition site; and
c) reacting the modified antibody of a) and the non-antibody domain of b) in the presence of KalbTG or a functionally active variant thereof and under conditions conducive to the activity of KalbTG, thereby forming a isopeptide bond between the first and the second recognition site, thus conjugating the modified antibody to the therapeutic entity.

13. The covalent conjugate of claim 9 or 10 or 11 or the covalent conjugate produced according to the method of claim 12 for use as a medicament.

14. The covalent conjugate of claim 9 or 10 or 11 or the covalent conjugate produced according to the method of claim 12 for use in treating a neurological disease or a brain disease, such as Alzheimer's disease or Parkinson's disease.

15. The covalent conjugate of claim 9 or 10 or 11 or the covalent conjugate produced according to the method of claim 12 for use in treating cancer, such as breast cancer.
